Europäisches Patentamt

(19) European Patent Office

Office européen des brevets

(11) EP 0 966 977 A2

## (12) EUROPEAN PATENT APPLICATION

(43) Date of publication:
29.12.1999 Bulletin 1999/52

(21) Application number: 99108656.2

(22) Date of filing: 14.05.1999

(51) Int. Cl.⁶: **A61L 15/34**, A61L 15/48,
A61L 15/58, A61F 13/15,
D04H 1/64, C09J 9/00,
C09J 201/00, C09J 11/00

(84) Designated Contracting States:
AT BE CH CY DE DK ES FI FR GB GR IE IT LI LU
MC NL PT SE
Designated Extension States:
AL LT LV MK RO SI

(30) Priority: 26.06.1998 EP 98111859
22.09.1998 EP 98117904

(71) Applicant:
THE PROCTER & GAMBLE COMPANY
Cincinnati, Ohio 45202 (US)

(72) Inventors:
• Muscat, Andreas
65824 Schwalbach (DE)
• Palumbo, Gianfranco
61348 Bad Homburg (DE)
• Corzani, Italo
66100 Chieti (IT)

(74) Representative:
Canonici, Jean-Jacques et al
Procter & Gamble,
European Service GmbH
65823 Schwalbach am Taunus (DE)

(54) **Hygienic article comprising hydrophilic adhesive having low surfactant release**

(57) This application relates to hygienic articles comprising two components which are at least partially joined to each other by means of an hydrophilic adhesive having low surfactant release into the acquired liquid. In particular, the adhesive has a contact angle with synthetic urine of less than 50° and reduces the surface tension of synthetic urine by less than 10mN/m five hours after immersion. The hygienic article according to the present invention exhibits sustained liquid handling performance even at high loads.

EP 0 966 977 A2

Printed by Xerox (UK) Business Services
2.16.7/3.6

**Description**

1. FIELD OF THE INVENTION

This application relates to hygienic articles which are intended to be used in direct contact or in close proximity to the skin of the user. More particularly, the present invention relates to hygienic articles comprising two components which are at least partially joined to each other by means of an oil resistant, hydrophilic adhesive.

2. BACKGROUND OF THE INVENTION

Hygienic articles are typically used in direct contact or in close proximity to the skin of the user. Whilst having a wide variety of purposes such as absorption of body exudates (e.g. diapers), covering the skin of the user (e.g. wound dressings), or the like, hygienic articles do face some common performance requirements which result from their exposure to the skin of the user.

Depending on the area of the body they are placed against, hygienic articles must be able to cope with differing amounts of water based liquids. While essentially all regions of the skin perspire and evaporate water, some hygienic articles have to deal with much higher amounts of liquids such as body exudates. To be able to move the liquid away from the skin of the user most hygienic articles comprise at least one liquid pervious component which is placed towards the skin of the user during the intended use.

During manufacture of hygienic articles of the above type the components including the liquid pervious component are generally joined to each other by means of an adhesive. Many commercially available adhesives are hydrophobic and hence are detrimental for the wettability and hence the liquid acquisition performance of hygienic articles.

It has been suggested, for example in WO 96/13283 (Chihani), to use hydrophilic adhesives for the construction of hygienic articles. Hydrophilic adhesives are typically obtained by blending surfactants into the adhesive composition as is described for example in WO 97/48779 (Gibes).

These prior art hydrophilic adhesives, however, release at least part of the surfactant into the liquid acquired into the hygienic article. In turn, the surface tension of the liquid is reduced by surfactant molecules migrating to the surface of the liquid. As a result, the capillary suction generated by the hygienic article during acquisition of the liquid which depends on the surface tension of the liquid is significantly reduced and the liquid acquisition rate drops.

It is an object of the present invention to provide a hygienic article which overcomes the limitation of the prior art hygienic articles.

It is a further object of the present invention to provide a hygienic article which is manufactured using a hydrophilic adhesive with low surfactant release into the acquired liquid.

It is a further object of the present invention to provide a hygienic article wherein the hygienic article further comprises an effective amount of a lotion coating which is partially transferable to the user's skin.

It is a further object of the present invention to provide a hygienic article wherein the hygienic article is a disposable absorbent article. Preferably the first component of the disposable absorbent article is a topsheet, more preferably comprising apertures of at least $0.2mm^2$. Alternatively, the first component of the disposable absorbent article of the present invention is a fecal management member.

It is a further object of the present invention to provide a disposable absorbent article wherein the second component is an acquisition layer or an absorbent core.

3. SUMMARY OF THE INVENTION

The present invention provides a hygienic article comprising a first component being liquid pervious, a second component, and an adhesive whereby at least part of the surface of the first component is joined to at least part of the surface of the second component by means of the adhesive. The adhesive of the hygienic article of the present invention has a Contact Angle with Jayco synthetic urine of less than 50° and reduces the surface tension of Jayco synthetic urine by less than 10mN/m five hours after immersion.

It is another aspect of the present invention to provide a process for manufacturing a hygienic article according to the present invention. This process comprises a step of applying the adhesive to the first component and/or the second component by an application technique selected from the group of glue beads, spiral gluing, spray gluing, curtain coating, slot coating, or microfiber meltblowing.

4. DETAILED DESCRIPTION OF THE INVENTION

This application relates to hygienic articles comprising two components which are at least partially joined to each other by means of an adhesive.

4.1 Hygienic Article

The term "hygienic article" as used herein refers to articles which are intended to be used in contact with or in close proximity to the skin of the user. Hygienic articles include absorbent articles such as absorbent pads, diapers, training pants, adult incontinence devices, sanitary napkins, bed pads, and the like. In addition, hygienic articles comprise articles of low or zero absorbency such as wound dressings, and the like. Hygienic articles of the present invention may be intended for multiple use or they may be disposable. The term "disposable" is used herein to describe hygienic articles which are not intended to be laundered or otherwise restored or reused as an hygienic article after a single use.

At least the first component of the hygienic article of the present invention is liquid pervious to allow liquid transfer to at least part of the interface between first component and second component.

4.2 Liquid Handling

Generally spoken, liquid transport in the hygienic article of the present invention can be achieved by two mechanisms:

1. "Free flow" whereby gravity is the driving force. This type of flow is enhanced by large open pores, and low surface energies, i.e. hydrophilic surfaces. However, this flow is per definition only in the direction of the gravity - which sometimes might be useful, but more often it is a strong constraint for accommodating for example different use situations such as position of the wearer etc.

2. "Capillary flow" whereby capillary forces dominate. This flow mechanism allows to overcome the gravity dominated flow. One of the equations used to describe the capillary flow is the Laplace equation

$$P_c = \frac{2\gamma\cos\theta}{R} \tag{1}$$

where $P_c$ is the capillary pressure, $\gamma$ is the surface tension of the liquid, $\theta$ is the contact angle between the liquid and the surface of the capillary, and $R$ is the radius of the capillary.

It can readily be seen from equation (1), that in order to increase the capillary pressure which is the driving force behind capillary flow, one needs to

- lower the contact angle of the liquid with the surface of the capillary by maximizing the difference between the surface energy of the capillary and the surface tension of the liquid
- provide small capillaries
- increase the surface tension of the liquid to be transported, and/or eliminate instances where the surface tension of the liquid is reduced

4.3 Hydrophilic Adhesive Having Low Surfactant Release

The hygienic article of the present invention comprises an adhesive which is deployed to at least partially join a surface of the first, liquid pervious component to a surface of the second component.

As used herein, the term "joined" encompasses configurations whereby a component is directly secured to another component by affixing the component directly to the other component. The term "partially joined" as used herein refers to configurations in which only parts of the respective surfaces are joined to each other, such as by being joined only at certain points, lines, areas, and the like. Preferably, the adhesive used to join the first and the second component is applied at a basis weight of at least 2 grams per square meter to those interface areas at which the first and the second component are joined to each other. More preferably, the adhesive is applied at a basis weight of at least 4 grams per square meter.

As can be seen from the Laplace equation (1) above, it is desirable to use an adhesive having a low contact angle with water, most preferably a contact angle of 0°. A low contact angle is desired to not impair wettability of at least the first component of the hygienic article.

It is further desirable to use an adhesive having a low surfactant release, most preferably no surfactant release, into the acquired liquid in order to not decrease the surface tension of the acquired liquid. A low surfactant release into the acquired liquid is desired to not reduce the capillary pressure of the hygienic article during acquisition of the liquid.

A variety of adhesives with different properties have been tested for the contact angle with Jayco synthetic urine and for the surface tension reduction of Jayco synthetic urine solution. The synthetic urine used in the test methods is commonly known as Jayco SynUrine and is available from Jayco Pharmaceuticals Company of Camp Hill, Pennsylvania, USA. The formula for the synthetic urine is: 2.0 g/: of KCl; 2.0 g/l of Na2SO4; 0.85 g/l of (NH4)H2PO4; 0.15 g/l (NH4)H2PO4; 0.19 g/l of CaCl2; ad 0.23 g/l of MgCl2. All of the chemicals are of reagent grade. The pH of the synthetic Urine is in the range of 6.0 to 6.4.

The results of the experiments are shown in Table 1.

Table I

| Experimental results for different adhesives. Adhesives 1 and 2 are hydrophilic adhesive compositions according to the present invention. Adhesive 3 is a commercially available hydrophobic adhesive. Adhesives 4 through 6 are comparative examples of commercially available hydrophilic adhesives. | | | |
| --- | --- | --- | --- |
| # | Adhesive | θ [°] | $\Delta\gamma$ 1h/5h after immersion [mN/m] |
| 1 | Fuller 1462 with 1% w/w Tegotain D | 30 | 4.9/8.8 |
| 2 | Fuller 1462 with 0.5% w/w Tegotain D | 40 | 2.2/6.0 |
| 3 | Fuller 1462 | 90 | 0/0 |
| 4 | Findley HX 2542 | 30 | 19.0/24.8 |
| 5 | Findley HX 2541 | 50 | 12.1/12.9 |
| 6 | NS 34-5652 | 30 | 17.3/>17.3 |
| Fuller 1462 is available from H.B. Fuller Company of St. Paul, Minnesota, USA. Tegotain D is availbale from Th. Goldschmidt AG of Essen, Germany. Findley HX 2542 and HX 2541 are available from Ato Findley Nederlands B.V. of Roosendaal, The Netherlands. NS 34-5652 is available from National Starch and Chemical Company of Neustadt/Weinstrasse, Germany. | | | |

### 4.3.1 Hydrophilicity

An adhesive suitable for the hygienic article of the present invention has a contact angle with Jayco synthetic urine of less than 50°, preferably less than 40°, more preferably less than 30°.

The hydrophilicity of an adhesive can be quantified by the Adhesive Contact Angle Measurement described hereinafter.

The following experimental procedure is used to make adhesive films to be used in measuring the contact angle of the adhesive.

(1) The adhesive is melted. The temperature to which the adhesive is heated should be chosen with care such that the melting procedure has substantially no detrimental effect on the adhesive. A temperature range of 100°-160°C has found to be useful for some adhesives.

(2) The adhesive is slot coated onto uncoated paper at a thickness of about 0.125 millimeters (5 mil). As a substrate, the uncoated side of a release paper is used. Such a release paper is available from 4P Rube Göttingen GmbH, of Göttingen, Germany, under the designation of "Rubesil Release Paper HV63-473 (einseitig beschichtet)".

Alternative methods to provide a film of adhesive of substantially uniform thickness of about 0.125 millimeters can also be used. In particular adjustments of the melting temperature have to be made according to the specific adhesive to be tested.

A method and apparatus for determining the contact angle of a droplet of Jayco synthetic urine on a film surface (such as prepared by the procedure outlined above) is described in U.S. patent No. 5,268,733. An apparatus to carry out this measurement is commercially available from TANTEC Inc. of Schaumburg, Illinois, USA, under the designation CONTACT ANGLE METER Model CAM-FILM.

During the measurement care has to be taken that a potential surfactant release from the adhesive into the synthetic urine does not reduce the surface tension of the synthetic urine. Such a surface tension reduction would lead to a reduced contact angle of the synthetic urine with the adhesive sample. One possibility to avoid surfactant release is to carry out the measurement immediately after placing the droplet of Jayco synthetic urine by imaging the droplet with a high speed video system and by subsequently measuring the contact angle on the video image.

### 4.3.2 Surfactant Release

An adhesive suitable for the present invention has a low surfactant release. As a consequence, the surfactant hence remaining within the adhesive composition renders that adhesive permanently hydrophilic. Such a permanently hydrophilic adhesive is able to substantially maintain its contact angle with distilled water even after exposure to aqueous liquids.

An adhesive suitable for the hygienic article of the present invention reduces the surface tension of Jayco synthetic urine by less than 10mN/m five hours after immersion into the synthetic urine, preferably by less than 9mN/m five hours after immersion, more preferably by less than 8mN/m five hours after immersion, even more preferably by less than 7mN/m five hours after immersion, most preferably by less than 6mN/m five hours after immersion.

The surfactant release of an adhesive into synthetic urine can be quantified by measuring the surface tension reduction of the synthetic urine in contact with the adhesive. Surfactant molecules may or may not migrate into the synthetic urine, thereby reducing the surface tension of the water. To carry out this measurement, an adhesive sample of 2 millimeters * 1.35 centimeters * 3 centimeters is placed in a beaker containing 100 milliliters of Jayco synthetic urine at ambient conditions (20°C room temperature, 50% relative humidity). In case the amount of adhesive to be tested is limited, the size of the adhesive sample and the volume of the liquid may be scaled down such that the same adhesive concentration is obtained. In case the adhesive sample is provided in a different geometric configuration, care has to be taken that the ratio of adhesive surface accessible to urine to the volume is substantially equal to the above sample configuration. Then, the surface tension reduction of the synthetic urine is measured after 5 hours. An exemplary apparatus to measure the surface tension of the water is commercially available from Krüss AG of Hamburg, Germany, under the designation "Interfacial Tensiometer K8".

### 4.3.3 Adhesive Composition

An adhesive suitable for the hygienic article of the present invention may be obtained by blending a low level of a suitable surfactant into a suitable hydrophobic adhesive. Blending of the adhesive may be achieved by weighing the hydrophobic adhesive and the surfactant into a container, carefully heating the container to a temperature slightly above the melting point of the hydrophobic adhesive, stirring for approximately 10 minutes, and finally cooling down the mixture.

The specific concentration range of the surfactant depends on the specific adhesive and the specific surfactant use as is obvious to the skilled practitioner of the art. A too low surfactant concentration leads to a high contact angle of the adhesive with water, i.e. to a more hydrophilic adhesive. With a too high concentration of surfactant, the release of surfactant molecules into the liquid is increased. A surfactant concentration that has been found to be useful for the present invention is between 0.1% and 3% by weight, preferably between 0.2% and 2% by weight, more preferably between 0.25% and 1.5% by weight, most preferably between 0.5% and 1% by weight.

A hydrophobic adhesive that has been found suitable for the present invention is commercially available from H. B. Fuller Company of St. Paul, Minnesota, USA under the designation 1462. A surfactant that has been found suitable for the present invention is commercially available from Th. Goldschmidt AG of Essen, Germany, under the designation Tegotain D. The surfactant concentration that has been found useful for this specific combination of adhesive and surfactant is between 0.5% and 1% by weight.

It will, however, be apparent to the skilled practitioner of the art that many other combinations of adhesives and surfactants also satisfy the requirements of the present invention. This is particularly true for adhesive and surfactant belonging to the same chemical classes as the aforementioned examples. The present invention is intended to also cover these combinations.

### 4.3.4 Adhesive Types

In order to allow efficient manufacture of the hygienic article of the present invention, hot melt adhesives are preferred for the present invention. The term "hot melt adhesive" as used herein refers to adhesives which are liquefied by heating prior to application, then are applied to the components in the liquid state, and reversibly solidify upon cooling down.

In some applications, it may be useful to deploy a pressure sensitive adhesive for the present invention. The term "pressure sensitive adhesive" as used herein refers to an adhesive that is tacky at room temperature.

### 4.3.5 Adhesive Configuration

The adhesive of the present invention may be configured in any form that compatible with the functionality of the respective hygienic article. Many different configurations and respective applications techniques such as spray gluing, spiral gluing, slot gluing, curtain coater gluing, glue beats, glue lines, reticulated networks, microfibers, and the like are well known in the art and are described for example in US-A- 3,911,173 (Sprague), US-A- 4,573,986 (Minetola), US-A-4,773,903 (Weisman), US-A- 4,785,996 (Ziecker), US-A- 5,418,009 (Raterman), US-A- 5, 436,066 (Chen), and US-A- 5,462,538 (Korpman). It will be readily apparent to the skilled practitioner of the art which adhesive configuration is to be chosen for a given application.

### 4.4 Other Components

The hygienic articles may comprise further components as is obvious to the skilled practitioner of the art. The first component and the second component according to the present invention may have different functional roles within the hygienic article.

### 4.5 Absorbent Article

A particularly preferable embodiment of the hygienic article of the present invention is the disposable absorbent article.

As used herein, the term "absorbent article" refers to devices which absorb and contain body exudates, and more specifically, refers to devices which are placed against the skin of the user to absorb and contain the various exudates discharged from the body of the user. Examples of disposable absorbent articles include feminine hygiene garments such as sanitary napkins and pantiliners, diapers, adult incontinence devices, diaper holders, training pants, and the like.

Disposable absorbent articles typically comprise a liquid pervious topsheet, a liquid impervious backsheet joined to the topsheet and an absorbent core positioned between the topsheet and the backsheet. Disposable absorbent articles and components thereof, including the topsheet, backsheet, absorbent core, and any individual layers of these components, have a body surface and a garment surface. As used herein, "body surface" means that surface of the article or component which is intended to be worn toward or adjacent to the body of the user, while the "garment surface" is on the opposite side and is intended to be worn toward or placed adjacent to the user's body or undergarments when the disposable absorbent article is worn.

In one embodiment of the absorbent article of the present invention, the first component is a topsheet. The term "topsheet" refers to a component of the absorbent article that is at least partially in direct contact with the skin of the user at least during some time of the intended use. The topsheet is at least partially joined to a second component by means of hydrophilic adhesive according to the present invention. The topsheet may be at least partially joined to an acquisition/distribution layer as the second component. Alternatively, the topsheet may be at least partially joined to an absorbent core as the second component. In both cases, the hydrophilic adhesive is used to enhance the liquid transfer from the topsheet to the second component.

The first component of the absorbent article of the present invention may also be a fecal management member being at least partially joined to the second component of the hygienic article. The fecal management member may be joined to an absorbent core as the second component. Fluid contact between the fecal management member and absorbent core underneath promotes the immobilization of the feces inside the fecal management member by dewatering the feces.

The first component of the absorbent article of the present invention may also be a batt of cellulosic or other fibers being comprised in the absorbent core of the absorbent article. In this case, the second component may be a plurality of superabsorbent polymer particulates which are dispersed through the absorbent core and are attached to the fibers by means of the adhesive of the present invention. In this case, microfibers have been found to be an effective adhesive configuration.

The following description generally discusses the absorbent core, topsheet, and backsheet materials that are useful in disposable absorbent articles.

### 4.5.1 Absorbent Core

In general, the absorbent core is capable of absorbing or retaining liquids (e.g., menses, urine, and/or other body exudates). The absorbent core is preferably compressible, conformable, and non-irritating to the user's skin. The absorbent core may be manufactured in a wide variety of sizes and shapes (e.g., rectangular, oval, hourglass, "T" shaped, dog bone, asymmetric, etc.). In addition to the absorbent composites of the present invention, the absorbent core may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these.

The configuration and construction of the absorbent core may also be varied (e.g., the absorbent core may have varying caliper zones and/or have a profile so as to be thicker in the center; hydrophilic gradients; gradients of the absorbent composite of the present invention, superabsorbent gradients; or lower average density and lower average basis weight zones, e.g., acquisition zones; or may comprise one or more layers or structures). The total absorbent capacity of the absorbent core should, however, be compatible with the design loading and the intended use of the absorbent article. Further, the size and absorbent capacity of the absorbent core may be varied to accommodate different uses such as diapers, incontinence pads, pantiliners, regular sanitary napkins, and overnight sanitary napkins, and to accommodate users ranging from infants to adults.

The absorbent core can include other absorbent components that are often used in absorbent articles, for example, a dusting layer, a wicking or acquisition layer, or a secondary topsheet for increasing the user's comfort.

4.5.2 Topsheet

The topsheet is preferably compliant, soft feeling, and non-irritating to the user's skin. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The topsheet of the absorbent article may further comprise an skin care composition which is at least partially transferable to skin of the user. The hygienic article may additionally comprise an skin care composition which is at least partially transferable to the skin of the user during the intended use. Preferably, such an skin care composition is positioned on at least a part of the user facing surface of the topsheet. The skin care composition may also be deployed in such a way that it is only released at the time of intended use such as being microencapsulated. Suitable skin care compositions are well known in the art and are described for example in WO 96/16682 (Roe et al.).

The topsheet 24 preferably has a plurality of apertures with an effective aperture size of at least 0.2 square millimetres, more preferably, the plurality of apertures have an effective aperture size of at least 0.5 square millimetres, even more preferably, the plurality of apertures have an effective aperture size of at least 1.0 square millimetres, and most preferably, the plurality of apertures have an effective aperture size of at least 2.0 square millimetres. Effective apertures are those which have a grey level of 18 or less on a standard grey level scale of 0-255, under the image acquisition parameters described in WO 94/28843 (Roe).

The topsheet 24 preferably has an effective open area of at least 15 percent, more preferably the topsheet has an effective open area of at least 20 percent, even more preferably, the topsheet has an effective open area of at least 25 percent, and most preferably the topsheet has an effective open area of at least 30 percent.

A method to determine the effective apertures size and the open area of the topsheet is given in WO 94/28843 (Roe).

4.5.3 Backsheet

The backsheet is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, or composite materials such as a film-coated nonwoven material. A suitable backsheet is a polyethylene film having a thickness of from about 0.012 mm (0.5 mil) to about 0.051 mm (2.0 mils). Exemplary polyethylene films are manufactured by Clopay Corporation of Cincinnati, Ohio, under the designation P18-1401 and by Tredegar Film Products of Terre Haute, Indiana, under the designation XP-39385. The backsheet is preferably embossed and/or matte finished to provide a more clothlike appearance. Further, the backsheet may permit vapors to escape from the absorbent core (i.e., the backsheet is breathable) while still preventing exudates from passing through the backsheet. The size of the backsheet is dictated by the size of the absorbent core and the exact absorbent article design selected.

4.5.4 Construction

The backsheet and the topsheet are positioned adjacent the garment surface and the body surface, respectively, of the absorbent core. The absorbent core is preferably joined with the topsheet, the backsheet, or both in any manner as is known by attachment means (not shown in Figure 3) such as those well known in the art. However, embodiments of the present invention are envisioned wherein portions of the entire absorbent core are unattached to either the topsheet, the backsheet, or both.

For example, the backsheet and/or the topsheet may be secured to the absorbent core or to each other by a uniform continuous layer of adhesive, a patterned layer of adhesive, or an array of separate lines, spirals, or spots of adhesive. The attachment means will preferably comprise an open pattern network of filaments of adhesive as is disclosed in U.S. Patent 4,573,986, issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zwieker, et al. on November 22, 1978; and U.S. Patent 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art.

The absorbent article of the present invention may be manufactured via a wide variety of processes well known in the art. During such a process the adhesive of the present invention is applied to the absorbent article by an application technique selected from the group of glue beads, spiral gluing, spray gluing, curtain coating, slot coating, or microfiber meltblowing as described above.

4.5.5 Other Preferable Embodiments

Another preferable embodiment of the absorbent article of the present invention are diapers. As used herein, the term "diaper" refers to an absorbent article generally worn by infants, and incontinent persons that is worn about the lower torso of the user. In other words, the term "diaper" includes infant diapers, training pants, adult incontinence devices, etc.

Diapers of the present invention can have a number of well known configurations, with the absorbent cores thereof being adapted to the present invention. Exemplary configurations are described generally in U.S. Patent 3,860,003 issued to Buell on January 14, 1975; U.S. Patent 5,151,092 issued to Buell et al. on September 29, 1992; U.S. Patent 5,221,274 issued to Buell et al. on June 22, 1993.

Another preferable embodiment of the absorbent article of the present invention are training pants. The term "training pants", as used herein, refers to disposable garments having fixed sides and leg openings. Training pants are placed in position on the user by inserting the user's legs into the leg openings and sliding the training pant into position about the user's lower torso. Suitable training pants are disclosed in U.S. Patent No. 5,246,433, issued to Hasse, et al. on September 21, 1993.

Another preferable embodiment of the absorbent article of the present invention are incontinence articles. The term "incontinence article" refers to pads, undergarments (pads held in place by a suspension system of same type, such as a belt, or the like), inserts for absorbent articles, capacity boosters for absorbent articles, briefs, bed pads, and the like regardless of whether they are worn by adults or other incontinent persons. Suitable incontinence articles are disclosed in U.S. Patent No. 4,253,461 issued to Strickland, et al. on March 3, 1981; U.S. Patent Nos. 4,597,760 and 4,597,761 issued to Buell; the above-mentioned U.S. Patent No. 4,704,115; U.S. Patent No. 4,909,802 issued to Ahr, et al.; U.S. Patent No. 4,964,860 issued to Gipson, et al. on October 23, 1990; and in U.S. Patent Application Serial No. 07/637,090 filed by Noel, et al. on January 3, 1991 (PCT Publication No. WO 92/11830 published on July 23, 1992).

**Claims**

1.   A hygienic article comprising a first component being liquid pervious, a second component, an adhesive, at least part of the surface of said first component being joined to at least part of the surface of said second component by means of said adhesive characterized in that said adhesive has a contact angle with distilled water of less than 50° when tested according to the contact angle test procedure described herein reduces the surface tension reduction of synthetic urine by less than 10mN/m five hours after immersion when tested according to the surface tension reduction test procedure described herein.

2. A hygienic article according to any of the preceding claims, wherein said hygienic article is a disposable absorbent article.

3. A disposable absorbent article according to Claim 2, wherein said first component is a topsheet.

4. A disposable absorbent article according to Claim 3, wherein said second component is an acquisition layer or an absorbent core.

5. A disposable absorbent article according to Claim 3, wherein said topsheet comprises apertures of at least 0.2 $mm^2$.

6. A disposable absorbent article according to Claim 2, wherein said first component is a fecal management member.

7. A disposable absorbent article according to Claim 6, wherein said second component is an absorbent core.

8. A process for manufacturing a hygienic article according to Claim 1, wherein said adhesive is applied to the first component and/or the second component by an application technique selected from the group of glue beads, spiral gluing, spray gluing, curtain coating, slot coating, microfiber meltblowing.